# EUROPÄISCHE PATENTSCHRIFT

(11) **EP 0 306 847 B1**
(45) Veröffentlichungstag und Bekanntmachung des Hinweises auf die Patenterteilung: **15.12.1993**
(21) Anmeldenummer: 88114316.8
(22) Anmeldetag: 02.09.1988
(51) Int. Cl.: C12N 9/22, C12P 19/34

(54) **Typ II-Restriktionsendonuklease Ksp632I**
Type II restriction endonuclease Ksp632I
Endonucléase de restriction du type II

(30) Priorität: 09.09.1987 DE 3730247; 03.08.1988 DE 3826389
(43) Veröffentlichungstag der Anmeldung: 15.03.1989
(73) Patentinhaber: BOEHRINGER MANNHEIM GMBH, 68298 Mannheim (DE)
(72) Erfinder: Bolton, Bryan John, Dr., Shirley Southampton (GB); Jarsch, Michael, Dr. rer. nat., D-8000 München 70 (DE); Gudrun, Schmitz, Dr. rer. nat., D-8139 Bernied (DE); Kessler, Christoph, Dr. rer. nat., D-8000 München (DE)

(56) Entgegenhaltungen:
- DE-A- 3 512 435
- GENE, Band 66, Nr. 1, 15. Juni 1988, Seiten 31-43, Elsevier Science Publ. B.V.,Amsterdam, NL; B.J. BOLTON et al.: "Ksp6321, a novel class-IIS restrictionendonuclease from Kluyvera sp. strain 632 with the asymmetric hexanucleotiderecognition sequence: 5'-CTCTTC(N)1-3'; 3'-GAGAAG(N)4-5'"
- NUCLEIC ACIDS RESEARCH, Band 16, Supplement, 9. Mai 1988, Seiten r271-r313, IRLPress Ltd, Oxford, GB; R.J. ROBERTS: "Restriction enzymes and their
- isoschizomers"

## Beschreibung

Die Erfindung betrifft die neue Typ II-Restriktionsendonuklease Ksp632I, ein Verfahren zu ihrer Gewinnung und ihrer Verwendung.

Typ II-Restriktionsendonukleasen sind Endodesoxyribonukleasen, welche bestimmte DNA-Sequenzen zu erkennen und zu spalten vermögen. Dabei werden Phosphodiesterbrücken in der Zielsequenz hydrolisiert und zwar in jedem Polynukleotidstrang eine. Typ II-Restriktionsendonukleasen sind daher wertvoll für die Analyse von DNA-Molekülen. Es sind zwar bereits für zahlreiche DNA-Sequenzen spezifische Typ II-Restriktionsendonukleasen bekannt, jedoch besteht immer noch Bedarf an der Schaffung weiterer Typ II-Restriktionsendonukleasen die für DNA-Sequenzen spezifisch sind und die bisher von keiner der bekannten Restriktionsendonukleasen erkannt werden. Der Erfindung liegt daher die Aufgabe zu Grunde, eine neue Restriktionsendonuklease zur Verfügung zu stellen, welche eine bisher von keinem derartigen Enzym erkannte Sequenz spezifisch zu erkennen und zu spalten vermag.

Gelöst wird diese Aufgabe erfindungsgemäß durch eine Restriktionsendonuklease, welche gekennzeichnet ist, durch die Erkennungssequenz 
5'-CTCTTCN NNN-N-3'

3'-GAGAAGN-NNN N-5'
Hierbei bedeutet N: A, G, C oder T, wobei jeweils G mit C und A mit T im komplementären DNA-Strang korrespondiert.

Vorzugsweise weist dieses Enzym die durch Pfeil definierte Spaltstelle auf, jedoch sind auch andere Spaltstellen innerhalb dieser Sequenz möglich. Die erfindungsgemäße neue Typ II-Restriktionsendonuklease, die im folgenden als Ksp632I bezeichnet wird, hat ein Temperaturoptimum bei ca. 37°C. Das Enzym besitzt eine gute Aktivität zwischen pH 7,2 und pH 8,0 bei einer Konzentration eines einwertigen Kations bei ca. 70 mmol/l (für K-acetat ) in 10 mmol/l Mg-Acetat und 0,5 mmol/l Dithiothreitol Das pH-Optimum liegt bei ca. pH 7,5. Ein Enzym, das isoschizomer zu Ksp623I ist, ist nicht bekannt.

Die Erkennungssequenz läßt sich durch vollständigen Verdau der DNAs der Viren SV40 und Adeno 2, der Phagen Lambda, phiX174, den Phagenderivaten M13mp8 und den Plasmiden pBR322 und pBR328 bestätigen. Diese DNA-Moleküle werden mit Ksp632I behandelt.

Tabelle 1 zeigt einen Vergleich der experimentell beobachteten Schnittstellenspezifität mit einer computerermittelten Schnittstellenspezifität für ein Enzym, welches die Sequenz CTCTTC erkennt.

**Tabelle 1**

| DNA | Anzahl der experimentell bestimmten Schnittstellen | Anzahl der durch Computeranalyse bestimmte Schnittstellen | Experimentell bestimmte Fragmentlängen | Durch Computeranalyse bestimmte Fragmentlängen | Spaltpositionen ermittelt durch Computeranalyse |
|---|---|---|---|---|---|
| Sv40 | 1 | 1 | 5250 | 5243 | 4365 |
| phiX174 | 2 | 2 | 5000 | 4985 | 3746 |
| | | | 400 | 401 | 4143 |
| M13mp8 | 2 | 2 | 4900 | 4929 | 4074 |
| | | | 2300 | 2300 | 6370 |
| pBR322 | 2 | 2 | 2500 | 2559 | 2535 |
| | | | 1800 | 1804 | 4157 |
| PBR328 | 1 | 1 | 4900 | 4907 | 3037 |

Die Schnittstelle innerhalb der Erkennungssequenz des Enzyms läßt sich wie folgt feststellen: Das Plasmid pUC18 (Gene 33 (1985) 103-109) wird mit EcoRI oder SacI linearisiert. Das EcoRI-Fragment wird am 5-Ende mit T4-Polynucleotid-Kinase unter Verwendung von [ γ³²P]-ATP markiert. Das SacI-Fragment wird am 3′-Ende mit Terminaler Transferase und [α³²P]- ddATP ebenfalls ³²P-markiert. Nach einem weiteren Verdau werden die entstandenen Fragmente, welche entweder am EcoRI-Ende 5′-markiert oder SacI-Ende 3′-markiert sind, durch Agarosegel-Elektrophorese isoliert und aus dem Gel gereinigt.

Aliquote dieser Fragmente werden mit Ksp632I behandelt und gleichzeitig einer basenspezifischen chemischen Spaltung (Maxam und Gilbert, Methods in Enzymology 65, 1980, 499- 560) zur Sequenzierung unterworfen.

Die Analyse der Fragmente wird durch Sequenzgel Elektrophorese (5 % Polyacrylamid, 8 mol/l Harnstoff) und anschließende Autoradiographie durchgeführt. Die Interpretation der Ergebnisse wird analog Methods in Enzymology 65, 1980, 391 - 401 durchgeführt. Hierbei zeigt sich, daß Ksp632I zwischen Position 296 und 297 bzw. 690 und 691 der Sequenz des Vektors pUC18 mit folgender Spezifität schneidet: 
5'-CTCTTCG CTA-T
SacI- EcoRI
3'-GAGAAGC-GAT A
CTCTTCC GCT-T-3'
GAGAAGG-CGA A-5'
Damit ergibt sich als allgemeine Spezifität: 
5'-CTCTTCN NNN-N-3'
3'-GAGAAGN-NNN N-5'
Die experimentell bestimmte Anzahl der Schnittstellen ist identisch mit der Anzahl der Schnittstellen die durch Computeranalyse mit den verschiedenen DNAs für die Sequenz CTCTTC erhalten wurde (Tabelle 1). Zusätzlich wurden diese Daten noch mit den Tabellen aus Gene 10 (1980) 357-370, verglichen. Ein doppelter Verdau von phiX174 DNA mit HgiAI und Ksp632I sowie von SV40 DNA mit AvaII und Ksp632I bestätigen, daß die Spezifität CTCTTC ist.

Erfindungsgemäß wird Ksp632I gewonnen, in dem man Kluyvera spezies 632 (DSM 4196) züchtet und das Enzym aus den Zellen gewinnt. Zur Gewinnung können die üblichen biochemischen Aufreinigungsmethoden verwendet werden, wobei in den jeweils erhaltenen Fraktionen das Vorhandensein des Enzyms anhand der Spaltung seiner Erkennungssequenz leicht nachgewiesen werden kann. Als Substrat eignet sich beispielsweise Adenovirus 2 DNA. Die erhaltenen DNA Fragmente werden in Agarosegelen in den für die Fragmentauftrennung üblichen Puffersystemen in Gegenwart von Ethidiumbromid elektrophoretisch aufgetrennt.

Der für die Gewinnung des Enzyms verwendete Microorganismus Kluyvera spezies 632 wächst aerob in Merck Standard I Medium.

Kluyvera spezies 632 wurde bei der deutschen Sammlung von Mikroorganismen, Gesellschaft für biotechnologische Forschung mbH, Griesebachstr.8, 3400 Göttingen, BRD, hinterlegt und trägt die Hinterlegungsnummer DSM 4196. Die optimalen Wachstumsbedingungen sind bei 10 - 30°C pH 7,2 - 7,8. Die Verdoppelungszeit beträgt etwa 2 Stunden.

Das Enzym wird isoliert und gereinigt durch die üblichen chemischen und mechanischen Methoden, beispielsweise durch Hochdruckdispersion, Ultraschall oder enzymatischen Aufschluß. In einer bevorzugten Ausführungsform des erfindungsgemäßen Verfahrens werden die Zellen einem Überdruck von 5 bar ausgesetzt. Die hierbei entstandene Zellenmasse wird in Tris-HCl Puffer pH 8.0, welcher Protease-Inhibitoren enthält, resuspendiert. Anschließend werden die Zellen über eine French-Presse aufgeschlossen und mit Polymin und Ammoniumsulfat präzipitiert. Die weitere Reinigung des Überstands, welcher das Enzym enthält, wird vorzugsweise über Molekularsiebchromatographie, Ionentauscherchromatographie sowie Affinitätschromatographie durchgeführt. Beispiele für geeignete Molekularsiebe sind Ultrogel AcA34 (LKB) sowie Hydroxylapatit (Boehringer Mannheim GmbH).
Als Anionentauscher geeignet ist das unter dem Namen DEAE Sephadex (Pharmacia) erhältliche Produkt. Als Material für die Affinitätschromatographie geeignet ist beispielsweise Heparin-Sepharose CL-6B (Pharmacia). Aber auch andere Chromatographiematerialien, die dem Fachmann bekannt sind, sind geeignet.

Die folgenden Beispiele erläutern die Erfindung weiter.

### Beispiel 1

Kluyvera spezies 632 wird bei 30°C 20 Stunden gezüchtet und der späten logarithmischen bzw. stationären Phase geerntet. Als Kulturmedium wird Merck Standardmedium I verwendet.

Die Zellpaste (30 g Naßgewicht) wird in 3 Volumen Puffer A (50 mmol/l Tris-HCl, pH 8,0, 0,1 mmol/l EDTA, 7 mmol/l 2-Mercaptoethanol), welcher Proteaseinhibitoren enthält, resuspendiert. Anschließend werden die Zellen durch zweimalige Passage einer French-Presse bei 23.000 lb/inch² aufgeschlossen. Anschließend wird Polymin P zugegeben und der Niederschlag abgetrennt. Der Überstand wird anschließend durch Zugabe von 60 % (w/v) Ammoniumsulfat behandelt. Der hierbei entstandene Niederschlag wird in 15 ml Puffer B (40 mmol/l Tris-HCl, pH 8,0, 0,1 mmol/l EDTA, 7 mmol/l 2-Mercaptoethanol, 10 % (V/V) Glycerin) aufgelöst. Anschließend wird an einer Sephacryl AcA34-Säule fraktioniert, welche zuvor mit Puffer B, dem 0,5 mmol/l NaCl zugesetzt wird, equilibriert ist. Die aktive Fraktion wird gegen Puffer B dialisiert. Anschließend wird sie auf eine Hydroxylapatitsäule, welche mit Puffer B equilibriert wurde, aufgegeben. Zur Elution wird ein Gradient von 0 - 1,0 mmol/l NaCl in Puffer B verwendet. Ksp623I wird in der Fraktion zwischen 0,4 und 0,6 mmol/l NaCl gefunden. Die aktive Fraktion wird gegen Puffer B dialysiert und auf eine Heparin-Sepharose CL-6B-Säule, die mit Puffer B equilibriert wurde, aufgebracht. Zur Elution wird ein Gradient von 0 - 1,0 mmol/l NaCl in Puffer B angewendet. Ksp623I wird in der Fraktion, die zwischen 0,4 und 0,6 mmol/l NaCl erscheint, gefunden.

Die aktive Fraktionen werden zusammengegeben und gegen Lagerpuffer (20 mmol/l Tris-HCl, pH 8,0, 10 mmol/l 2-Mercaptoethanol, und 100 mmol/l NaCl, 0,1 mmol/l EDTA, 50 % (v/v) Glycerin) dialysiert.

### Beispiel 2

### Bestimmung der Aktivität

Definition der Enzymeinheiten: 1 U Ksp632I spaltet 1 µg Lambda-DNA innerhalb 1 Stunde bei 37°C in 25 µl Endvolumen.
Zu einer Mischung von 12 µl Inkubationspuffer (66 mmol/l Tris-acetat , pH 7,9/37°C, 20 mmol/l Magnesiumacetat , 132 mmol/l K-acetat , und 1 mmol/l Dithiothreitol werden 7 µl Wasser und 5 µl Lambda DNA (optische Dichte: 4 OD/ml) sowie 1 µl Ksp632I-Lösung (1U/µl) zugegeben. Die Lösung wird eine Stunde bei 37°C inkubiert, auf Eis gekühlt und mit 5 µl eines Abstopp-Reagenses, bestehend aus 7 mmol/l Harnstoff, 20 % (w/v) Saccharose, 60 mmol/l EDTA und 0,01 % (w/v) Bromphenolblau versetzt. Anschließend wird eine Trennung durch Elektrophorese in 1 % Agarose-Gelen für 3 - 4 Stunden bei 100 V durchgeführt. Die erhaltenen Banden werden über Vergleich mit einem DNA-Längenstandard identifiziert.

## Patentansprüche

1. Typ II-Restriktionsendonuklease gekennzeichnet durch die Erkennungssequenz, und die durch den Pfeil definierte Spaltungsstelle 
5'-CTCTTCN NNN-N-3'
3'-GAGAAGN-NNN N-5'
 darin bedeutet N: A, G, C oder T, wobei jeweils G mit C und A mit T im komplementären DNA-Strang korrespondiert.

2. Restriktionsendonuklease nach Anspruch 1 gekennzeichnet durch ein Temperatur-Optimum bei ca. 37°C und ein pH-Optimum zwischen 7,2 und 8,0.

3. Verfahren zur Gewinnung der Restriktionsendonuklease nach den Ansprüchen 1 oder 2, dadurch gekennzeichnet, daß man Kluyvera spezies 632 (DSM 4196) züchtet und das Enzym aus den Zellen gewinnt.

4. Verfahren nach Anspruch 3, dadurch gekennzeichnet, daß man die Zellen aufschließt und den Zellüberstand gewinnt.

5. Verfahren nach Anspruch 4, dadurch gekennzeichnet, daß man zur Feinreinigung den Zellüberstand einer Affinitätschromatographie, einer Molekularsiebchromatographie und einer abschließenden Chromatographie über einen Kationentauscher unterwirft.

6. Verfahren nach Anspruch 5, dadurch gekennzeichnet, daß man zur Affinitätschromatographie trägerfixiertes Heparin verwendet.

7. Verwendung der Restriktionsendonuklease nach Anspruch 1, zur Erkennung und Spaltung der komplementären doppelsträngigen DNA-Sequenz 5'-CTCTTCNNNNN-3' oder der komplementären doppelsträngigen DNA-Sequenz 5'-NNNNNGAAGAG-3'.

## Claims

1. Type II restriction endonuclease characterised by the recognition sequence and the cleavage position defined by the arrow 
5'-CTCTTCN NNN-N-3'
3'-GAGAAGN  NNN N-5'
 wherein N signifies: A, G, C or T, whereby, in each case, G corresponds with C and A with T in the complementary DNA strand.

2. Restriction endonuclease according to claim 1, characterised by a temperature optimum at about 37°C and a pH optimum between 7.2 and 8.0.

3. Process for the obtaining of restriction endonuclease according to claim 1 or 2, characterised in that one cultures Kluyvera species 632 (DSM 4196) and obtains the enzyme from the cells.

4. Process according to claim 3, characterised in that one digests the cells and obtains the cell supernatant.

5. Process according to claim 4, characterised in that, for the fine purification, one subjects the cell supernatant to an affinity chromatography, to a molecular sieve chromatography and to a subsequent chromatography over a cation exchanger.

6. Process according to claim 5, characterised in that, for the affinity chromatography, one uses carrier-fixed heptain.

7. Use of the restriction endonuclease according to claim 1 for the recognition and cleavage of the complementary double-stranded DNA sequence 5'-CTCTTCNNNNN-3' or of the complementary double-stranded DNA sequence 5'-NNNNNGAAGAG-3'.

## Revendications

1. Endonucléase de restriction de type II, caractérisée en ce qu'elle reconnaît la séquence ci-après, coupée de la façon indiquée par la flèche : 
5'-CTCTTCN NNN-N-3'
3'-GAGAAGN-NNN N-5'
 dans laquelle N représente l'une quelconque des bases A, G, C ou T, G correspondant à C et A à T sur le brin d'ADN complémentaire.

2. Endonucléase de restriction selon la revendication 1 caractérisée par une température optimale d'environ 37°C et un pH optimal situé entre 7,2 et 8,0.

3. Procédé de production de l'endonucléase de restriction selon la revendication 1 ou 2, caractérisé par la culture de Kluyvera espèce 632 (DSM 4196) et l'isolement de l'enzyme à partir d'un extrait cellulaire.

4. Procédé selon la revendication 3, caractérisé par la rupture des cellules et l'isolement du surnageant.

5. Procédé selon la revendication 4, caractérisé par la purification du surnageant par chromatographie d'affinité, chromatographie sur tamis moléculaire et, finalement, chromatographie échangeuse de cations.

6. Procédés selon la revendication 5, caractérisé par l'utilisation d'héparine fixée à un support pour la chromatographie d'affinité.

7. Utilisation de l'endonucléase de restriction selon la revendication 1, pour l'identification et la coupure de la séquence d'ADN double brin complémentaire 5'-CTCTTCNNNNN-3' ou de la séquence d'ADN double brin complémentaire 5'-NNNNNGAAGAG-3'.
